# EUROPEAN PATENT SPECIFICATION

(11) **EP 1 246 668 B1**
(45) Date of publication and mention of the grant of the patent: **30.11.2005**
(21) Application number: 00983475.5
(22) Date of filing: 25.08.2000
(51) Int. Cl.: A61P 25/06, A61K 31/48, A61K 31/42, A61K 31/4196, A61K 31/4045, A61K 31/138, A61K 9/19

(54) **AN RAPID ACTING FREEZE DIRED ORAL PHARMACEUTICAL COMPOSITION FOR TREATING MIGRAINE**
SCHNELL-WIRKENDE, GEFRIER-TROCKNETE, ORALE, PHARMAZEUTISCHE FORMULIERUNG FÜR DIE BEHANDLUNG VON MIGRÄNE
COMPOSITION PHARMACEUTIQUE LYOPHILISEE A ACTION RAPIDE S'ADMINISTRANT PAR VOIE ORALE UTILISEE POUR TRAITER LA MIGRAINE

(30) Priority: 01.12.1999 IN MA116099
(43) Date of publication of application: 09.10.2002
(73) Proprietor: Natco Pharma Limited, Hyderabad 500 033 (IN)
(72) Inventor: VENKATESWARA RAO, Pavuluri Manager R & D (F), Hyderabad 500 033 (IN); KHADGAPATHI, Podili Director- Technical, Hyderabad 500 033 (IN)
(74) Representative: Nachshen, Neil Jacob
(86) International application number: PCT/IN2000/000078
(87) International publication number: WO 2001/039836

(56) References cited:
- EP-A- 0 255 141
- EP-A- 0 774 253
- WO-A-96/15785
- WO-A-98/08490
- GB-A- 2 166 952
- US-A- 4 078 065
- US-A- 5 151 264

## Description

This invention relates to an improved fast acting oral pharmaceutical composition. The invention particularly relates to an improved fast-acting, freeze-dried pharmaceutical composition. The invention also relates to a process for preparing such a composition. The composition is intended for rapid systemic absorption of the active substance incorporated in the composition through oral mucosa, thereby eliminating the need for parenteral administration of the medicament for crisis management and is suitable for patients who have difficulty in swallowing solid dosage forms and/or are non cooperative in swallowing medication.

In the recent times numerous advances have taken place in the field of pharmacology and pharmaceutics with respect to the administration of drugs to treat various conditions. Despite the tremendous advancements in the field, however, drugs continue to be administered using substantially the same techniques that have been used for many decades. The vast majority of pharmaceutical agents continue to be administered either orally or by injection. Nevertheless, it is frequently found in the art that neither of these administration routes is effective in all cases, and both administration routes suffer from several disadvantages.

Oral administration is probably the most prevalent method of administering pharmacological medicaments. The medicament is generally incorporated into a tablet, capsule, or a liquid base, and then swallowed. The oral administration modality is often preferred because of its convenience. In addition, oral administration is generally non-threatening, painless, and simple to accomplish for most patients.

Nevertheless, oral administration of drugs suffers from several disadvantages. One disadvantage is that pediatric and geriatric patients frequently have difficulty swallowing pills and other solid dosage-forms, and such patients often refuse to cooperate in swallowing a liquid medication. In addition, for many medicaments, the act of swallowing the medicament often requires fluids that increase gastric volume and the likelihood of nausea and vomiting. This occurs more often in migraine patients.

A further problem with oral administration is that the rate of absorption of the drug substance into the bloodstream after swallowing varies from patient to patient. The absorption of the drug substance is dependent upon the movement of the drug substance from the stomach to the small and large intestines and the effects of secretions from these organs and on the resulting pH within the stomach and intestines. Anxiety and stress can dramatically reduce these movements and secretions, prevent or reduce the final effects of the drug substance, and delay onset of the drug's effects.

Most significant is the fact that there is normally a substantial delay between the time of oral administration and the time that the therapeutic effect of the drug begins. As mentioned above, the drug must pass through the gastrointestinal system in order to enter the bloodstream; which typically takes forty-five minutes or longer. As mentioned above, anxiety and stress often increase this delay. For many applications, such as pre-medication before surgery or where immediate relief from pain or a serious medical condition or immediate effectiveness of the drug is required, this delay is unacceptable.

An additional disadvantage of oral administration is that many drugs almost immediately experience metabolism or inactivation. The veins from the stomach and the small and large intestines pass directly through the liver. Thus, drugs entering the bloodstream must first pass through the liver before distribution into the general blood circulation. More than sixty percent of most drugs (and essentially one hundred percent of certain drugs) are removed from the patient's bloodstream during this "first pass" through the liver. The result is that oral administration is either impractical for many drugs, particularly many central nervous system and many cardiovascular-acting drugs that are used for rapid onset in critical care situations, as a pre-medication prior to surgery or for the induction of anesthesia, or requires very high doses when compared to the parenteral route of administration, ranging from 2.0 to 5.0 fold increase in the total dose administered in a day.

Further, additional stress is placed on the liver as it removes the excess drug from the bloodstream. This is particularly severe if the drug treatment has been occurring over an extended period of time. The liver may become overloaded with the drug's metabolite, which then must be excreted. As a result, there is an increased risk of hepatic or renal disorders.

Parenteral administration probably is used most, after the oral route for administering medicaments. However parenteral administration has many drawbacks. Parenteral administration is generally threatening, painful, and cumbersome to accomplish for most patients. In addition, the cost of therapy is more when administration is effected by the parenteral route.

Some investigators have suggested that it may be possible to administer medication through the buccal mucosa of the cheek pouch or by sublingual administration. Refer U.S. Pat. No. 4,671,953 entitled "METHODS AND COMPOSITIONS FOR NONINVASIVE ADMINISTRATION OF SEDATIVES, ANALGESICS, AND ANESTHETICS". Such administration through the mucosal tissues of the mouth, pharynx, and esophagus of therapeutic drugs possesses a distinct usefulness. Administration of drugs by this route does not expose the drug to the gastric and intestinal digestive juices. In addition, the drugs largely bypass the liver on the first pass through the body, avoiding additional metabolism and/or inactivation of the drug. Consequently the total dose of the drug required for eliciting the desired therapeutic response is considerably reduced in some instances.

Rapid dissolving or disintegrating pharmaceutical dosage forms are available for human patients who have difficulty in swallowing conventional dosage forms such as tablets or capsules and for the sublingual and buccal administration of drug. Most of these dosage forms are prepared either by compression or molding.

Compressed tablets are prepared after mixing the active ingredient with suitable excipients, into tablets, which disintegrate in 1 to 3 minutes into granules or fine particles when suspended in water. Moreover such dispersion in water invariably has to be swallowed and the drug has to dissolve in gastrointestinal fluids before being absorbed systemically.

Molded tablets are prepared by blending excipients with the drug, mixing with a solvent to make a workable mass, filling into the mold plate with sufficient pressure, ejecting the tablet in wet condition by placing on top of a plate which has projecting pegs and drying. As tablets dry, the solvent migrates to the surface and may carry the active ingredient or other soluble components to the tablet surface. This can produce a non-homogenous distribution of the drug throughout the tablet.

While administration of certain drugs through the oral mucosal tissues has show promise, development of a fully acceptable method for producing a medication in a desirable form and administering the medication has been elusive for long.

Many of the disadvantages of rapidly disintegrating compressed and molded tablets mentioned above can be over come by freeze-dried tablets. Freeze-dried or lyophilised dosage forms are generally known to dissolve rapidly or disintegrate when they come in contact with water or any aqueous fluids. These dosage forms include of an open matrix network of water - soluble or water dosage forms include of an open matrix network of water - soluble or water dispersible carrier material, which is impregnated with a unit dose of the pharmaceutical active agent. These dosage forms are prepared by first adding the pharmaceutical active to a solution comprising the carrier material and a suitable solvent, typically water. The resulting composition is then subjected to a freeze-drying procedure where by the solvent sublimes under high vacuum.

By the term "Rapid disintegrating" it is meant that the tablet disintegrates in mouth within 30 seconds, preferably the tablet disintegrates (dissolves or disperses) within 10 seconds or less.

By the term "Open matrix network" it is meant a network of water-soluble or water dispersible carrier material having interstices dispersed throughout. The open matrix network of carrier material is of generally low density. The density of the tablet may be affected by the amount of substance incorporated into the tablet. The open matrix network, which is similar in structure to solid foam, enables a liquid to enter the product through the interstices and permeate through the interior. Permeation by saliva in the mouth exposes the carrier material of both the interior and exterior of the product to the action of saliva where the net work of carrier material is rapidly disintegrated. The open matrix structure is of a porous nature and enhances disintegration of tablet as compared with ordinary tablets, pills and capsules. Rapid disintegration results in rapid release of any drug carried by the matrix.

In US Patent US 4 371 516 and GB 2 111 423 (Inventors: Gregory et. al.,) a process and composition of a shaped article carrying chemicals, pharmaceuticals which disintegrate rapidly in water, having an open matrix network of carrier material prepared by sublimation process using a mold was disclosed. Partially hydrolysed gelatin, hydrolysed dextran, dextrin, alginates, polyvinyl alcohol, polyvinylpyrrolidone or acacia were used as carrier materials for administration of pharmaceutical substances.

Seager has described a method for preparing rapid- dissolving dosage forms that disintegrate instantaneously releasing the drug which dissolves or disperses in the saliva [Seager, H., J Pharm. Pharmcol. 1998, 50(4), 375 - 382].

In EP 0 636 365 A1 a freeze dried pharmaceutical dosage form containing a porous matrix of a water soluble or water dispersible carrier material which comprises coated pharmaceutical particles was disclosed. The pharmaceutical granules were coated with a blend of water soluble and water insoluble polymers to overcome the bitter taste of the dosage form upon administration allowing the coated particles to be swallowed and is useful for acetaminophen, loperamide, famotidine or aspirin for patients who have difficulty in swallowing conventional tablets or capsules. However the release of drug from coated particles requires some time, which may delay the onset of action.

In US 5 785 989, a composition and methods of manufacture for producing a candy type flavored dissolvable matrix containing medicament capable of absorption through the mucosal tissues of the mouth, pharynx and esophagus useful in administering lipophilic and non lipophilic drugs to which an appliance or holder is attached was disclosed. The solid dosage form is meant for use in the delivery of drug in a pharmacologically effective dose in which the drug being dispersed in a soluble matrix capable of being sucked on while held in the mouth, with attached holder to permit convenient insertion and removal of the drug containing integral mass into and out of the mouth of the patient. The dissolvable matrix optionally contains permeation enhancers and pH buffering agents to increase the absorption of the drug through the mucosal tissue. However this hard to dissolve candy preparation is not suitable for administration to non-cooperative and unconscious patients.

In WO 9626714 a process for the preparation of a solid pharmaceutical dosage form comprising a carrier, selegiline hydrochloride as an active ingredient in the form of a fast dispersing dosage form designed to release the active ingredient in the oral cavity has been disclosed.

We continued our research work towards developing a composition for the treatment of migraine and associated symptoms considering (i) the requirement of non threatening, painless and simple administration of such drug to the patient especially to pediatric and geriatric patients (ii) the requirement of the onset of action to be rapid meaning that the absorption of the drug is effected through oral mucosa. (iii) requiring only reduced dosage of the drug and that (iv) the medicine can be taken without water.

With the information available in the prior art as explained above ,we directed our research work towards developing a pharmaceutical composition useful for the treatment of migraine and associated symptoms which composition can be administered orally and that the active ingredient disintegrates rapidly which in turn results in the rapid release of the drug . To our knowledge such a composition for the treatment of migraine is not hitherto known.

Accordingly, the main objective of the present invention is to provide an improved pharmaceutical composition useful for the treatment of migraine and associated symptoms, which can be administered orally.

Another objective of the present invention in to provide an improved fast-acting freeze-dried pharmaceutical composition for oral administration, useful for the treatment of migraine and associated symptoms, at reduced total dose of active substance required.

Yet another objective of the present invention is to provide an improved pharmaceutical composition for the treatment of migraine and the associated symptoms, which is capable of rapidly disintegrating in the mouth, carrying active substance(s) in the form of free base or its pharmaceutically acceptable salt or ester, thereby allowing the dissolution of the active substance in saliva and transmucosal absorption of the medicament for rapid onset of action.

Still another objective of the present invention is to provide a process for manufacture of such an improved pharmaceutical composition.

The present invention is based on our finding, as a result of sustained research carried out , that when an anti migraine drug administered orally in the form of a tablet with an open matrix and associated symptoms net work comprising water soluble or water dispersible carrier materials, synergises the drug resulting in the rapid onset of its action i.e., relief from migraine and other associated symptoms It is further observed that a lower dose of anti migraine drug i.e., 10.0 to 60.0 percent of the orally administered dose, is sufficient to elicit an equal therapeutic response when such a pharmaceutical composition is administered sublingually.

Accordingly, the invention provides an improved freeze dried pharmaceutical composition useful or the treatment of migraine and associated symptoms, which comprises at least one active ingredient selected from sumatriptan, zolmitriptan, and rizatriptan and a water soluble and water dispersible carrier material in an open matrix network, as herein defined , with or without co administered active substances and / or other exepients normally employed in such compositions, the resulting composition rapidly disintegrating, as herein defined, in the mouth.

Another feature of the invention is to provide a process for the preparation of the improved pharmaceutical composition explained above. The pharmaceutical composition of the present invention is prepared using freeze-drying or lyophilisation process generally known it the art. Such processes are described in U.S. patent Nos. 4,305,502 and 4,371,516 both to Gregory et al, U.S. Patent No.: 4,642,903 to Davies and by Seager [Seager, H., J. Pharm. Pharmcol. 1998, 50(4), 375 - 382].

Accordingly the present invention also provides a process for the preparation of an improved freeze dried pharmaceutical composition useful for the treatment of migraine and associated symptoms, which comprises adding one or more pharmaceutically active substances useful for the treatment of migraine or associated symptoms to a solution / suspension of a water soluble or water dispersible carrier material thereby forming an open matrix network , as herein defined and if necessary , adding other additives normally employed preparing in such compositions transferring the resultant solution / suspension to a mold of the desired shape and size of the final product and freezing the product in a freeze dryer at a temperature in the range of -50 to 10°C and further to drying at a temperature of -40 to 90°C under vacuum of in the range of 1.0 × 10⁻² to 7.5 × 10⁻¹ torr.

The additives, which may be employed in the pharmaceutical composition, may be selected from variety of substances . They are exemplified below. The additives may be organoleptic additives such as sweetening agents, flavoring agents, coloring agents; pharmaceutically acceptable preservatives, solublising agents; surface-active agents and/or buffering agents. The pharmaceutical composition also may contain other additives such as permeation enhancers, chelating salts and stablising agents.

The active substances that are incorporated in the pharmaceutical composition of the invention are those used for the treatment of migraine and associated symptoms such as sumatriptan, zolmitriptan, and rizatriptan.

Typical drugs, which can be administered by this means in combination with the above active substances and useful for the treatment of migraine associated symptoms include drugs such as antihistaminic and antiallergenic agents e.g. chlorpheniramine maleate, diphenhydramine, terphenidine, flunarizine, cetirizine; sympathomimetics e.g. phenylpropanolamine pseudoephedrine; anti emetics e.g. dimenhydrinate, domeperidone, ondansetron, granisetron, prochlorperazine metoclopropamide; analgesic and anti-inflammatory agents e.g. naproxen sodium, paracetamol etc.

The active substance may be present in the form of a base or pharmaceutically acceptable form of its salt or ester having good penetration / absorption through transmucosal layers in the oral cavity. The pharmaceutical composition may carry a combination of active substances intended for the treatment of migraine and the associated symptoms.

The active substance may be present in the composition in a therapeutically effective amount that can be readily determined by one skilled in the art. In determining such amounts, the particular compound being administered, the bioavailability characteristics of the pharmaceutical, the dose regime, the age and weight of the patient and other factors must be considered. For example for sumatritan the dose may range from 5.0 to 50.0mg preferably from 10.0 to 25.0mg.

The open net work of carrier materials which may be used in the pharmaceutical composition of the invention may be any water-soluble or water-dispersible inert pharmaceutical excipients capable of forming a rapidly disintegratable open matrix network, preferably a water soluble material since this results in most rapid disintegration of the matrix when the composition is placed in the mouth. Such carrier matrix network may be selected from polyvinyl pyrrolidone, polyvinyl alcohol, partially hydrolised gelatins, dextrins, alginates, carboxymethyl celluloses pectin's, hydroxyproyl cellulose, hydroxyethyl cellulose, methyl cellulose, carboxy vinyl polymers such as carbomer 934P and carbomer 974P. Other suitable carrier materials for inclusion in the matrix network include sugars such as dextrose, mannitol, lactose, galactose, cyclodextrins, inorganic salts such as sodium phosphate, sodium chloride and aluminum silicates. Preferred carrier materials include pharmaceutical grade gelatins, pectins (nonhydrolysed and partially hydrolysed); carboxy vinyl polymers; polyvinylpyrrolidone; polyvinyl alcohol; mannitol and mixtures thereof. The amount of such carrier matrix material in the pharmaceutical composition may range from 10.0 to 90.0 percent preferably 30.0 to 70.0 percent by weight on dry basis.

The pharmaceutical composition also may contain ingredients other than the active substance and carrier matrix forming material. These additional ingredients include buffering agents, organoleptic additives such as sweetening agents, flavoring agents, coloring agents, pharmaceutically acceptable preservatives, solubilising agents and/or surface-active agents. The pharmaceutical composition also may contain additives such as permeation enhancers, chelating salts and stablising agents.

It is desirable to include buffering agents in the composition. Buffering agents provide the ability to place the medication in the mouth in a favorable pH environment for passage across the mucosal tissues of the mouth, pharynx and esophagus. Buffering agents can be used to effect pH change in the salival environment of the mouth in order to favor the existence of a non-ionized form of the active substance, which more rapidly moves through the mucosal tissues. Appropriate pH adjustment can aid in producing a more palatable product with active substances which are either severely acidic (and thus sour) or severely basic (and thus bitter). A buffer system such as citric acid / sodium citrate or citric acid / disodium hydrogen ortho phosphate etc., may be used.

The amount of each organoleptic additive when used may range from 0.025 to 5.0 percent by weight on dry basis of the pharmaceutical composition. Preservatives may be added to the pharmaceutical composition as most of the ingredients promote microbial growth in presence of moisture. The preservatives and the concentrations used in the pharmaceutical composition are selected from among those known in art and approved for oral pharmaceutical dosage forms.

The composition may also contain surface-active agents such as polysorbate 80, sodium lauryl sulphate and the like to aid in the dispersion of the active substance in the solution / dispersion of the carrier material during manufacturing process and in saliva during administration to the patient. The amount of surface-active agent may range from 0.01 to 2.0% of the pharmaceutical composition on dried basis.

The permeability of both lipophilic and non-lipophilic drugs across the oral mucosal membrane may be improved by using suitable permeation enhancers. The permeation enhancers that optionally may be used in the pharmaceutical composition are bile salts such as sodium cholate, sodium glycocholate, sodium glycodeoxycholate, taurodeoxycholate, sodium deoxycholate, etc., sodium dodecyl sulphate, dimethylsulphoxide, sodium lauryl sulphate, salts and other derivatives of saturated and unsaturated fatty acids, bile salt analogs, derivatives of bile salts, surfactants, or such synthetic permeation enhancers as described in U.S. Pat. No. 4, 746, 508.

The permeation enhancer concentration within the freeze-dried pharmaceutical composition may be varied depending on the potency of the enhancer and the rate of dissolution of the active substance in saliva. A toxic effect to mucus membrane or irritation sets the upper limit for enhancer concentration.

Chelating salts and other stablising agents known in the art optionally may be employed in the pharmaceutical composition to enhance the stability of the active substance incorporated. Salts of citric acid, edetic acid etc., may be used as chelating salts, the amount of which may range from 0.005 to 2.0 percent by weight of pharmaceutical composition on dry basis. Cyclodextrins, hydroaypropyl methylcellulose and the like may be used as stablising agents.

The depression in the mould used in the process of making the composition may be significantly larger in which case the final product can be cut into the desired shape to the desired shape and sizes after the freeze-drying process is completed.

The molds may also be coated or lined for easy removal of the freeze-dried pharmaceutical composition. Preferred molds are made from polypropylene, polyvinyl chloride filled with talc and/or simethicone, aluminum with a layer of hydrogenated vegetable oil, silicone / simethicone baked onto the surface of the mold in contact with the solution/suspension. Blisters may also be used which may be made out of polyvinylchloride or polyvinyl dichloride coated polyvinylchloride or aluminum films.

To facilitate the dissolution / dispersion of the active ingredient in water to form the open matrix net work, a co-solvent such as ethyl alcohol, tert-butyl alcohol and/or solubilising agents such as cyclodextrins, lecithin and non-ionic surface-active agents e.g. sorbitan esters and polysorbates may be used. The amount of such co-solvent when used may range from 2.0 to 25.0 percent, more preferably from 5.0 to 15.0 percent of the total volume of solvent used in the process. The amount of solubilising agent when used ,may range from 0.1 to 5.0 percent, preferably 0.5 to 2.5 percent by weight of pharmaceutical composition on dry basis.

Optionally, the active substances are dissolved in water by incorporating a small quantity of acids such as citric acid, sulphuric acid, hydrochloric acid followed by pH adjustment to 4.5 to 6.5 with pharmaceutically acceptable buffering agents such as sodium citrate or disodium hydrogen ortho phosphate.

Once the freeze-drying process is complete, the freeze-dried pharmaceutical composition is transferred to a moisture-impervious packaging, such as blister pack. Alternately when the solution / suspension is freeze-dried within preformed blisters, the blisters are sealed with aluminum foil having a suitable sealing layer on the inner side. To remove the tablet from the blister, perforation is provided one side of the blister.

The invention is described in detail in the Examples given below which are provided by way of illustration only.

### EXAMPLE - 1

| Each tablet contains: | |
|---|---|
| Sumatriptan | 7.50 mg |
| Citric acid anhydrous | 1.68 mg |
| Disodium hydrogen ortho phosphate | 2.42 mg |
| Polyvinyl alcohol | 3.0 % w/w |
| Mannitol | 25.0 % w/w |
| Methyl paraben sodium | 0.1%w/w |
| Propyl paraben sodium | 0.01%w/w |

Sumatriptan is dissolved in purified water with the addition of a few drops of diluted sulphuric acid and added to a solution of polyvinyl alcohol, mannitol and methyl paraben sodium and propyl paraben sodium in water. Citric acid anhydrous and disodium hydrogen ortho phosphate are added in the form of a 0.05M solution to adjust the pH of the solution between 3.0 - 4.0. Purified water is added up to volume. The resultant solution is filtered and 0.30ml of this solution is transferred to a mold having depressions and freeze dried at a temperature of -20 to 40°C and vacuum of 1.0 x 10⁻² to 1.0 x 10⁻¹ torr. The freeze-dried tablets are collected into a moisture resistant container.

### EXAMPLE - 2

| Each tablet contains: | |
|---|---|
| Sumatriptan | 15.0 mg |
| Citric acid anhydrous | 1.68 mg |
| Disodium hydrogen ortho phosphate | 2.42 mg |
| Polyvinyl pyrrolidone | 1.0 % w/w |
| Partially hydrolysed gelatin | 1.0 % w/w |
| Mannitol | 75.0 % w/w |
| Methyl paraben sodium | 0.1 % w/w |
| Propyl paraben sodium | 0.01%w/w |
| Pineapple flavour | 0.1 % w/w |

Sumatriptan is dissolved in purified water with the addition of a few drops of diluted sulphuric acid and added to a solution of polyvinyl pyrrolidone, partially hydrolysed gelatin, mannitol, pineapple flavour and methyl paraben sodium and propyl paraben sodium in water. Citric acid anhydrous and disodium hydrogen ortho phosphate are added in the form of a 0.05M solution to adjust the pH of the solution between 3.0 - 4.0. Purified water is added up to volume. The resultant solution is filtered and 0.30ml of this solution is transferred to preformed blisters and freeze dried at a temperature of -20 to 40°C and vacuum of 1.0 x 10⁻² to 1.0 x 10⁻¹ torr. The freeze-dried tablets are collected into a moisture resistant container.

### EXAMPLE - 3

| Each tablet contains: | |
|---|---|
| Sumatriptan Succinate | 14.0 mg |
| Disodium hydrogen ortho phosphate | 2.42 mg |
| Gelatin partially hydrolysed | 3.0 % w/w |
| Mannitol | 50.0 % w/w |
| Methyl paraben sodium | 0.1 % w/w |
| Propyl paraben sodium | 0.01%w/w |
| Pineapple flavour | 0.1 % w/w |
| Straw berry flavour | 0.05%w/w |

Sumatriptan succinate is dissolved in purified water. Disodium hydrogen ortho phosphate is added in the form of a 0.05M solution to adjust the pH of the solution between 4.0 - 5.5. This solution is added to a solution of partially hydrolysed gelatin. Mannitol, strawberry flavour, pineapple flavour and methyl paraben sodium and propyl paraben sodium are dissolved in the solution. Purified water is added up to volume. The resultant solution is filtered and 0.30ml of this solution is transferred to a mold containing depressions and freeze dried at a temperature of -20 to 40°C and vacuum of 1.0 x 10⁻² to 1.0 x 10⁻¹ torr. The freeze-dried tablets are collected into a moisture resistant container.

### EXAMPLE-4

| Each tablet contains: | |
|---|---|
| Sumatriptan succinate | 14.00 mg |
| Ondansetron hydrochloride dihydrate | 5.0 mg |
| Citric acid anhydrous | 1.68 mg |
| Disodium hydrogen ortho phosphate | 2.42 mg |
| Polyvinyl alcohol | 3.0 % w/w |
| Mannitol | 25.0 % w/w |
| Methyl paraben sodium | 0.1 % w/w |
| Propyl paraben sodium | 0.01%w/w |

Sumatriptan succinate and ondansetron hydrochloride dihydrate are dissolved in purified water and added to a solution of polyvinyl alcohol, mannitol and methyl paraben sodium and propyl paraben sodium in water. Citric acid anhydrous and disodium hydrogen ortho phosphate are added in the form of a 0.05M solution to adjust the pH of the solution between 3.0 - 4.0. Purified water is added up to volume. The resultant solution is filtered and 0.30ml of this solution is transferred to a mold containing depressions and freeze dried at a temperature of -20 to 40°C and vacuum of 1.0 x 10⁻² to 1.0 x 10⁻¹ torr. The freeze-dried tablets are collected into a moisture resistant container.

### EXAMPLE - 5

| Each tablet contains: | |
|---|---|
| Zolmitriptan | 7.50 mg |
| Citric acid anhydrous | 1.68 mg |
| Disodium hydrogen ortho phosphate | 2.42 mg |
| Gelatin, partially hydrolysed | 3.0 % w/w |
| Mannitol | 25.0 % w/w |
| Methyl paraben sodium | 0.1 % w/w |
| Propyl paraben sodium | 0.01%w/w |

Zolmitriptan is dispersed in purified water: Citric acid anhydrous and disodium hydrogen ortho phosphate are added in the form of a 0.05M solution to adjust the pH of the suspension between 4.0 - 5.5. This suspension is added to a solution of partially hydrolysed gelatin in which mannitol, methyl paraben sodium and propyl paraben sodium are dissolved. Purified water is added up to volume. The resultant dispersion is transferred in 0.30ml quantities to a mold containing depressions and freeze dried at a temperature of -20 to 40°C and vacuum of 1.0 x 10⁻² to 1.0 x 10⁻¹ torr. The freeze-dried tablets are collected into a moisture resistant container.

### EXAMPLE - 6

| Each tablet contains: | |
|---|---|
| Zolmitriptan | 7.50 mg |
| Ondansetron hydrochloride dihydrate | 5.0 mg |
| Citric acid anhydrous | 1.68 mg |
| Disodium hydrogen ortho phosphate | 2.42 mg |
| Polyvinyl alcohol | 3.0 % w/w |
| Mannitol | 25.0 % w/w |
| Methyl paraben sodium | 0.1%w/w |
| Propyl paraben sodium | 0.01%w/w |

Zolmitriptan and ondansetron hydrochloride dihydrate are dispersed in purified water and added to a solution of polyvinyl alcohol, mannitol and methyl paraben sodium and propyl paraben sodium in water. Citric acid anhydrous and disodium hydrogen ortho phosphate are added in the form of a 0.05M solution to adjust the pH of the solution between 3.0 - 4.0. Purified water is added up to volume. The resultant dispersion is transferred in 0.30ml quantities to a mold containing depressions and freeze dried at a temperature of -20 to 40°C and vacuum of 1.0 x 10⁻² to 1.0 x 10⁻¹ torr. The freeze-dried tablets are collected into a moisture resistant container.

### EXAMPLE - 7

| Each tablet contains: | |
|---|---|
| Ergotamine tartrate | 1.0 mg |
| Prochlorperazine maleate | 2.5 mg |
| Mannitol | 25.0 % w/w |
| Methyl paraben sodium | 0.1 % w/w |
| Propyl paraben sodium | 0.01 %w/w |
| Gelatin | 1.0 % w/v |

Gelatin is dissolved in water and is partially hydrolysed by autoclaving for 2 hours at 121°C and 15 1b pressure. Mannitol, ergotamine tartrate and prochlorperazine maleate are dissolved in 1% w/v gelatin solution (partially hydrolysed) containing methyl paraben sodium and propyl paraben sodium as preservatives. Purified water is added up to volume. The resultant solution is filtered and 0.30ml of this solution is transferred to a mold containing depressions and freeze dried at a temperature of -20 to 40°C and vacuum of 1.0 x 10⁻² to 1.0 x 10⁻¹ torr. The freeze-dried tablets are collected into a moisture resistant container.

### EXAMPLE-8

| Each tablet contains: | |
|---|---|
| Sumatriptan | 10.0mg |
| Citric acid anhydrous | 1.68 mg |
| Disodium hydrogen ortho phosphate | 2.42 mg |
| Carbomer 934P | 0.5 % w/w |
| Mannitol | 25.0 % w/w |
| Methyl paraben sodium | 0.1 % w/w |
| Propyl paraben sodium | 0.01%w/w |

Sumatriptan is dispersed in purified water and added to a suspension of carbomer 934P in which mannitol and methyl paraben sodium and propyl paraben sodium were dissolved. Citric acid anhydrous and disodium hydrogen ortho phosphate are added in the form of a 0.05M solution to adjust the pH of the suspension between 4.0 to 5.0. Purified water is added up to volume. The resultant suspension is transferred in 0.30ml quantities to a preformed polyvinyl dichloride coated polyvinyl chloride blister and freeze dried at a temperature of -20 to 40°C and vacuum of 1.0 x 10⁻² to 1.0 x 10⁻¹ torr. The freeze-dried tablets are sealed in the blister using aluminum foil.

### Advantages of the invention:

The invention has the advantage of
1) rapid onset of action due to the rapid absorption of the active substance through oral mucosa.
2) reduced dosage of the drugs as absorption through oral mucosa bypasses the first-pass metabolism and over comes possible degradation in the gastrointestinal tract.
3) easy to administer to pediatric and geriatric patients.
4) medicament can be taken without water.

## Claims

1. An oral pharmaceutical composition useful for the treatment of migraine comprising:
at least one active agent selected from the group consisting of sumatriptan, zolmitriptan and rizatriptan and pharmaceutically acceptable salts or esters thereof; and
a water soluble or dispersible carrier in the form of an open matrix network,
wherein the composition is a freeze dried composition capable of rapidly disintegrating in a subject's mouth and delivering the active agent to the subject by absorption through oral mucosa.

2. The pharmaceutical composition of claim 1, further comprising at least one selected from the group consisting of antihistaminic, antiallergenic, sympathomimetic, antiemetic, analgesic and anti-inflammatory agents.

3. The pharmaceutical composition of claim 1, further comprising at least one selected from the group consisting of chlorpheniramine maleate, diphenhydramine, terphenidine, flunarizine, cetrizine; phenylpropanolamine, pseudoephedrine, dimenhydrinate, domeperidone, ondansetron, granisetron, prochlorperazine metoclopropamide, naproxen sodium, and paracetamol.

4. The pharmaceutical composition of any preceding claim wherein the carrier comprises at least one selected from the group consisting of polyvinyl pyrrolidone, polyvinyl alcohol, partially hydrolysed gelatins, dextrins, alginates, carboxymethyl celluloses, pectins, hydroxyproyl cellulose, hydroxyethyl cellulose, methyl cellulose, carboxy vinyl polymers, sugars, cyclodextrins and inorganic salts.

5. The pharmaceutical composition of any preceding claim wherein the carrier comprises at least one selected from the group consisting of pharmaceutical grade gelatins, pectins (nonhydrolysed and partially hydrolysed); carboxy vinyl polymers; polyvinylpyrrolidone; polyvinyl alcohol; mannitol and mixtures thereof.

6. The pharmaceutical composition of any preceding claim wherein the amount of carrier material employed ranges from 10.0 to 90.0 percent by weight of the composition on a dry basis.

7. The pharmaceutical composition of any preceding claim wherein the amount of carrier material employed ranges from 30.0 to 70.0 percent by weight of the composition on a dry basis.

8. The pharmaceutical composition of any preceding claim further comprising at least one selected from the group consisting of organoleptic additives, buffering agents and and-microbial preservatives.

9. The pharmaceutical composition of any preceding claim further comprising a solubilising agent in an amount of 0.1 to 5.0 percent of the composition by weight on a dry basis.

10. The pharmaceutical composition of any preceding claim further comprising a solubilising agent in an amount of 0.5 to 2.5 percent of the composition by weight on a dry basis.

11. The pharmaceutical composition of any preceding claim further comprising a penetration enhancer selected from the group consisting of bile salts and analogs and derivatives thereof, surfactants and saturated and unsaturated fatty acids salts and derivatives thereof.

12. The pharmaceutical composition of any preceding claim further comprising at least one selected from the group consisting of sodium cholate, sodium glycocholate, sodium glycodeoxycholate, taurodeoxycholate, sodium deoxycholate, sodium dodecyl sulphate, docusate sodium and sodium lauryl sulphate.

13. A method of making the pharmaceutical composition of any preceding claim, comprising:
forming a solution or dispersion of the active agent and the carrier;
transferring the solution or dispersion to a mold;
freezing the solution or dispersion in the mold; and
drying the frozen product.

14. The method of claim 13, wherein the freezing is carried out at a temperature of -50 to 10 degrees C.

15. The method of claim 13, wherein the drying is carried out at a temperature of -40 to 90 degrees C and a pressure of 1 x 10⁻² to 7.5 x 10⁻¹ torr.

16. Use of a pharmaceutical composition according to any of claims 1 to 12 for the manufacture of a medicament for treating migraine.

## Patentansprüche

1. Oral einzunehmende pharmazeutische Zusammensetzung, die zur Behandlung von Migräne nützlich ist, Folgendes umfassend:
mindestens einen Wirkstoff, der aus der Gruppe ausgewählt ist, die aus Sumatriptan, Zolmitriptan und Rizatriptan und deren pharmazeutisch zugelassenen Salzen und Estern besteht; und
einen wasserlöslichen oder wasserdispergierbaren Träger in Form eines Netzes mit offener Matrix,
wobei die Zusammensetzung eine gefriergetrocknete Zusammensetzung ist, die in der Lage ist, sich im Mund einer Person schnell aufzulösen und den Wirkstoff durch Absorption durch die Mundschleimhaut an die Person abzugeben.

2. Pharmazeutische Zusammensetzung nach Anspruch 1, darüber hinaus mindestens einen Stoff umfassend, der aus der Gruppe ausgewählt ist, die aus Antihistaminika, Antiallergika, Sympathomimetika, Antiemetika, Analgetika und Entzündungshemmern besteht.

3. Pharmazeutische Zusammensetzung nach Anspruch 1, darüber hinaus mindestens einen Stoff umfassend, der aus der Gruppe ausgewählt ist, die Chlorpheniraminmaleat, Diphenhydramin, Terphenidin, Flunarizin, Cetrizin, Phenylpropanolamin, Pseudoephedrin, Dimenhydrinat, Domeperidon, Ondansetron, Granisetron, Prochlorperazin, Metoclopropamid, Naproxennatrium und Paracetamol besteht.

4. Pharmazeutische Zusammensetzung nach jedem vorhergehenden Anspruch, wobei der Träger mindestens einen Stoff umfasst, der aus der Gruppe ausgewählt ist, die aus Polyvinylpyrrolidon, Polyvinylalkohol, teilweise hydrolysierten Gelatinen, Dextrinen, Alginaten, Carboxymethylcellulosen, Pektinen, Hydroxypropylcellulose, Hydroxyethylcellulose, Methylcellulose, Carboxyvinylpolymeren, Zuckern, Cyclodextrinen und anorganischen Salzen besteht.

5. Pharmazeutische Zusammensetzung nach jedem vorhergehenden Anspruch, wobei der Träger mindestens einen Stoff umfasst, der aus der Gruppe ausgewählt ist, die aus Gelatinen in pharmazeutischer Qualität, Pektinen (nicht hydrolysiert und teilweise hydrolysiert); Carboxyvinylpolymeren; Polyvinylpyrrolidon; Polyvinylalkohol; Mannitol und Gemischen davon besteht.

6. Pharmazeutische Zusammensetzung nach jedem vorhergehenden Anspruch, wobei die Menge des eingesetzten Trägerstoffs 10 bis 90 Gew.-% der Zusammensetzung auf Trockenbasis beträgt.

7. Pharmazeutische Zusammensetzung nach jedem vorhergehenden Anspruch, wobei die Menge des eingesetzten Trägerstoffs 30 bis 70 Gew.-% der Zusammensetzung auf Trockenbasis beträgt.

8. Pharmazeutische Zusammensetzung nach jedem vorhergehenden Anspruch, darüber hinaus mindestens einen Stoff umfassend, der aus der Gruppe ausgewählt ist, die aus organoleptischen Zusatzstoffen, Puffersubstanzen und antimikrobiellen Konservierungsmitteln besteht.

9. Pharmazeutische Zusammensetzung nach jedem vorhergehenden Anspruch, darüber hinaus einen Lösungsvermittler in einer Menge von 0,1 bis 5,0 Gew.% der Zusammensetzung auf Trockenbasis umfassend.

10. Pharmazeutische Zusammensetzung nach jedem vorhergehenden Anspruch, darüber hinaus einen Lösungsvermittler in einer Menge von 0,5 bis 2,5 Gew.-% der Zusammensetzung auf Trockenbasis umfassend.

11. Pharmazeutische Zusammensetzung nach jedem vorhergehenden Anspruch, darüber hinaus einen Penetrationsverstärker umfassend, der aus der Gruppe ausgewählt ist, die aus Gallensalzen und deren Entsprechungen und Derivaten, Tensiden und gesättigten und ungesättigten Fettsäuresalzen und deren Derivaten besteht.

12. Pharmazeutische Zusammensetzung nach jedem vorhergehenden Anspruch, darüber hinaus mindestens einen Stoff umfassend, der aus der Gruppe ausgewählt ist, die Natriumcholat, Natriumglycocholat, Natriumglycodeoxycholat, Taurodeoxycholat, Natriumdeoxycholat, Natziumdodecylsulfat, Docusat-Natrium und Natriumlaurylsulfat besteht.

13. Verfahren zum Herstellen der pharmazeutischen Zusammensetzung nach jedem vorhergehenden Anspruch, Folgendes umfassend:
Ausbilden einer Lösung oder Dispersion aus dem Wirkstoff und dem Träger;
Übertragen der Lösung oder Dispersion auf eine Form;
Gefrieren der Lösung oder Dispersion in der Form; und
Trocknen des gefrorenen Erzeugnisses.

14. Verfahren nach Anspruch 13, wobei das Gefrieren bei einer Temperatur von -50 bis 10°C erfolgt.

15. Verfahren nach Anspruch 13, wobei das Gefrieren bei einer Temperatur von -40 bis 90°C und einem Druck von 1 x 10⁻² bis 7,5 x 10⁻¹ Torr erfolgt.

16. Verwendung einer pharmazeutischen Zusammensetzung nach einem der Ansprüche 1 bis 12 zur Herstellung eines Medikaments zur Behandlung von Migräne.

## Revendications

1. Composition pharmaceutique orale utile pour le traitement de la migraine comprenant :
au moins un agent actif choisi dans le groupe constitué par le sumatriptan, le zolmitriptan et le rizatriptan et leurs sels ou esters pharmaceutiquement acceptables ; et
un support soluble ou dispersable dans l'eau sous la forme d'un réseau de matrice ouvert ;
dans lequel la composition est une composition lyophilisée capable de se désintégrer rapidement dans la bouche d'un sujet et de libérer l'agent actif au sujet par absorption par la muqueuse buccale.

2. Composition pharmaceutique selon la revendication 1, comprenant en outre au moins un élément choisi dans le groupe constitué par les agents antihistaminiques, les antiallergiques, les sympathomimétiques, les antiémétiques, les analgésiques et les anti-inflammatoires.

3. Composition pharmaceutique selon la revendication 1, comprenant en outre au moins un élément choisi dans le groupe constitué par le maléate de chlorphéniramine, la diphénhydramine, la terphénidine, la flunarizine, la cétrizine, la phénylpropanolamine, la pseudoéphédrine, le diménhydrinate, la dompéridone, l'ondansétron, le granisétron, la prochlorpérazine métoclopropamide, le naproxen sodium, et le paracétamol.

4. Composition pharmaceutique selon l'une quelconque des revendications précédentes dans laquelle le support comprend au moins un élément choisi dans le groupe constitué par la polyvinylpyrrolidone, l'alcool de polyvinyle, les gélatines partiellement hydrolysées, les dextrines, les alginates, les carboxyméthylcelluloses, les pectines, l'hydroxypropylcellulose, l'hydroxyéthylcellulose, la méthylcellulose, les carboxyvinylpolymères, les sucres, les cyclodextrines et les sels inorganiques.

5. Composition pharmaceutique selon l'une quelconque des revendications précédentes dans laquelle le support comprend au moins un élément choisi dans le groupe constitué par les gélatines de grade pharmaceutique, les pectines (non hydrolysées et partiellement hydrolysées) ; les carboxyvinylpolymères ; la polyvinylpyrrolidone ; l'alcool de polyvinyle et des mélanges de ceux-ci.

6. Composition pharmaceutique selon l'une quelconque des revendications précédentes, dans laquelle la quantité de matériau support utilisé est de 10,0 à 90,0 pour cent en poids de la composition sur une base sèche.

7. Composition pharmaceutique selon l'une quelconque des revendications précédentes, dans laquelle la quantité de matériau de support utilisé est de 30,0 à 70,0 pour cent en poids de la composition sur une base sèche.

8. Composition pharmaceutique selon l'une quelconque des revendications précédentes comprenant en outre au moins un élément choisi dans le groupe constitué par les additifs organoleptiques, les agents tampons et les conservateurs antimicrobiens.

9. Composition pharmaceutique selon l'une quelconque des revendications précédentes, comprenant un agent solubilisant, dans une quantité de 0,1 à 5,0 pour cent de la composition en poids sur une base sèche.

10. Composition pharmaceutique selon l'une quelconque des revendications précédentes, comprenant en outre un agent solubilisant dans une quantité de 0,5 à 2,5 pour cent de la composition en poids sur une base sèche.

11. Composition pharmaceutique selon l'une quelconque des revendications précédentes, comprenant un stimulateur de pénétration choisi dans le groupe constitué par les sels biliaires et leurs analogues et dérivés, les tensio-actifs et les sels d'acides gras saturés et insaturés et leurs dérivés.

12. Composition pharmaceutique selon l'une quelconque des revendications précédentes, comprenant au moins un élément choisi dans le groupe constitué par le cholate de sodium, le glycocholate de sodium, le glycodéoxycholate de sodium, le taurodéoxycholate, le déoxycholate de sodium, le sodium dodécylsulfate, le docusate sodium, et le laurylsulfate de sodium.

13. Procédé de réalisation de la composition pharmaceutique selon l'une quelconque des revendications précédentes, comprenant :
la formation d'une solution ou dispersion de l'agent actif et du support ;
le transfert de la solution ou dispersion dans un moule ;
la congélation de la solution ou dispersion dans le moule ; et
la dessiccation du produit congelé.

14. Procédé selon la revendication 13, dans lequel la congélation est réalisée à une température de -50 à 10°C.

15. Procédé selon la revendication 13, dans lequel la dessiccation est réalisée à une température de -40 à 90°C et une pression de 1 x 10⁻² à 7,5 x 10⁻¹ torr.

16. Utilisation d'une composition pharmaceutique selon l'une quelconque des revendications 1 à 12, dans la fabrication d'un médicament pour traiter la migraine.
